# EUROPEAN PATENT APPLICATION

(11) **EP 1 130 105 A1**
(43) Date of publication of application: **05.09.2001**
(21) Application number: 00200726.8
(22) Date of filing: 01.03.2000
(51) Int. Cl.: C12N 15/82, C12N 15/74, C12N 15/80, C12N 15/81, A01H 5/00

(54) **Transformation of eukaryotic cells by mobilizable plasmids**

(71) Applicant: Rijksuniversiteit te Leiden, 2312 AV Leiden (NL); Stichting Binair Vector Systeem, 2343 RS Oegstgeest (NL)
(72) Inventor: Hooykaas, Paul Jan Jacob, 2343 RS Oegstgeest (NL); Escudero, Jesus, 2318 MJ Leiden (NL)
(74) Representative: Ottevangers, Sietse Ulbe

(57) **Abstract**

The invention relates to the transfer of genetic material to eukaryotic cells by means of a process resembling conjugation, in particular by a system partially based on Agrobacterium tumefaciens-like transfer systems. In particular transfer of genetic material into plant cells is disclosed using mobilisable, but non-conjugative plasmids by means of an Agrobacterium virulence system. The invention provides a method for transferring genetic material which is not a typical T-DNA surrounded by Agrobacterium T-borders from an Agrobacterium virulence system to a eukaryotic host cell, comprising providing said genetic material on a mobilisable plasmid, capable of forming a relaxosome, bringing said mobilisable plasmid in an Agrobacterium having at least the activity of the transfer genes of Agrobacterium not present on said mobilisable plasmid, whereby the necessary gene products providing the same or similar activity as a functional VirB operon are also present and cocultivating said agrobacterium with said eukaryotic host cell.

## Description

The invention relates to the transfer of genetic material to eukaryotic cells by means of a process resembling conjugation, in particular by a system partially based on Agrobacterium tumefaciens-like transfer systems. In particular transfer of genetic material into plant cells is disclosed using mobilisable, but non-conjugative plasmids by means of an Agrobacterium virulence system.

The Agrobacterium virulence system is routinely used for the transfer of genetic material into plants. Indications have been obtained that this system mediates the transfer of said genetic material by a process which resembles conjugation. Conjugation is a sophisticated process which requires a complex set of sequences and gene products present in bacteria in order to be successful.

Naturally, only genetic material which is surrounded by the Ti border repeats (T-DNA) is transferred by the Agrobacterium virulence system. The only exception is that the promiscuous IncQ plasmid can be transferred by the Agrobacterium system. The frequency of this transfer however is 100-fold less than that of the natural T-DNA. Moreover it depends on the presence of many, if not all of the activities of the Agrobacterium system.

The present invention now provides a new group of plasmids which can be transferred by the Agrobacterium virulence system at an efficiency at least similar to that of the natural T-DNA.

Thus the invention provides a new group of plasmids which comprise the mobilisation functions necessary for the transfer of genetic material to eukaryotic cells, but which need some but not all functions, determined by an

Agrobacterium virulence system i.e. that of A. tumefaciens or a related species.

Thus in one embodiment the invention provides a method for transferring genetic material by means of an Agrobacterium virulence system to a eukaryotic host cell, providing said genetic material on a mobilisable plasmid, capable of forming a relaxosome, bringing said mobilisable plasmid in an Agrobacterium having at least the activity of the transfer genes of Agrobacterium not present on said mobilisable plasmid, whereby the necessary gene products providing the same or similar activity as a functional VirB operon are also present and cocultivating said Agrobacterium with said eukaryotic host cell. According to the present invention a mobilisable plasmid is defined as a plasmid that has (preferably in cis or optionally with some functions in trans) the capability of forming a relaxosome (in a suitable surrounding such as Agrobacterium) and being capable of being transferred by an Agrobacterium vir-like system into eukaryotic cells.

The necessary and desired functions will be discussed in detail in the detailed description.

The genetic material to be transferred into the eukaryotic host cell (plant, yeast, fungi or animal) may be any genetic material of interest ranging from genes to antisense or cosuppressing sequences, etc. The field of providing cells with additional genetic material is by now well ploughed and candidate sequences are well within the skill of the art. Typically the transfer will occur by a conjugation-like system based on an Agrobacterium-like system. Any such system will suffice if it is capable of complementing the functions lacking on the mobilisable plasmid. Typically it will be necessary to provide for physical contact between the eukaryotic host cell and the Agrobacterium-like vehicle in order to effect transfer. Herein this is referred to as cocultivation. Typical functions to be present on the mobilisable plasmids according to the invention include the origin of transfer or mobilisation, herein referred to as *oriT.* Thus in another embodiment the invention provides a method whereby said mobilisable plasmid comprises a functional *oriT*. Preferably the mobilisable plasmid also has the virD-like mobilization functions necessary for relaxosome formation and a virD-like coupling factor for connecting the relaxosome to the VirB transport channel but these latter functions may also be provided in trans. Also preferred is a method whereby the mobilisable plasmid comprises itself a functional VirB operon. Functional virD sequences and virB operons and other sequences encoding functional products are defined herein as sequences encoding products having at least one the same or similar relevant activity as e.g. the virD products, although their actual physical structures may differ. Preferred of course are derivatives of these functional products such as virD themselves or homologues thereof in different species. Derivatives may include functional fragments of e.g. virD.

It is of course preferable if the mobilisable plasmids according to the invention can be easily propagated and/or manipulated. The invention in a preferred embodiment thus provides a method whereby said mobilisable plasmid is derived from a group of mobilisable plasmids present in enterobacteria, which plasmids are non-self-conjugative, more preferably a method wherein said group of mobilisable plasmids comprises small plasmids which can be maintained in high copy number in enterobacteria, in particular wherein said group of enterobacteria comprises E.coli.

The exemplified and preferred plasmid according to the invention is derived from the mobilisable plasmid CloDF13.

The invention in another preferred embodiment provides a method wherein said mobilisable plasmid is produced and or multiplied in an enterobacterium, preferably E.coli.

The invention of course also provides the plasmids according to the invention themselves and their uses.

Thus, in one embodiment the invention provides a mobilisable plasmid comprising genetic material to be transferred into a eukaryotic cell by Agrobacterium transfer, said mobilisable plasmid further comprising an *ori*T sequence, but whereby the mobilization functions and coupling factor are provided in trans from another replicon.

In yet another embodiment the invention provides a mobilisable plasmid comprising genetic material to be transferred into a eukaryotic cell by Agrobacterium transfer, said mobilisable plasmid further comprising a functional *oriT* and sequences encoding functional mobilisation products and a coupling factor. The requirements and desirabilities of the presence of the several functions in cis and/or in trans has been touched upon before and is discussed in greater detail in the detailed description. Although not necessarily so, it is preferred to have virB-like activity *in trans,* just as virD-like activities.

The plasmids according to the inventions can be put to uses according to the invention, in particular the use of transferring genetic material to cells, in particular the nucleus or cell organelles. The plasmids according to the invention typically are well suited for such sophisticated uses or can be manipulated to fit such uses.

Of course the preferred cell to be provided with additional genetic material according to the invention is a plant cell. The invention thus also includes plant cells and plants or parts of plants and/or offspring of plants or gametes of plants comprising plasmids or remains of plasmids or genetic material originating from plasmids according to the invention. The invention will be described in more illustrative detail in the following detailed description.

In still another preferred embodiment the invention provides a mobilisable plasmid comprising 2 *oriT* sequences preferably flanking a nucleic acid to be transferred thereby allowing transfer of the area between the 2 *ori*Ts, separate from the rest of the mobilisable plasmid.

### Detailed description.

The natural trans-kingdom genetic transfer from *Agrobacterium tumefaciens* to plants during tumorigenesis represents a sophisticated process of bacterial colonization (for review, see Hooykaas and Schilperoort, 1992). Such an infection relies on the transfer of a precise DNA fragment (the T-DNA) which is flanked by two 25-bp directly repeated sequences (the T-DNA borders). The T-DNA is part of a large bacterial tumor-inducing plasmid (the pTi) and is exported presumably as a DNA/protein complex (the T-complex) from the bacterial cell directly into the plant cell, where it integrates into the plant genome and expresses its *onc*-genes giving plant-cell divisions resulting in crown-gall tumor formation. *Agrobacterium*-mediated transformation has been shown as well for yeasts and fungi (Bundock *et al.*, 1995; de Groot *et al.*, 1998) and the mechanism of T-DNA transfer resembles the one previously observed in plants.

The genetic requirements for T-DNA transfer to plants have been extensively studied: a large set of *vir*-genes located adjacent to the T-DNA in the Ti plasmid are involved in this. Besides it requires the presence in *cis* of at least one of the 25-bp border repeats, the so-called right border (RB) (for reviews see Hooykaas and Beijersbergen, 1994; Sheng and Citovsky, 1996). Via the VirA protein the bacteria detect specific plant metabolites, such as acetosyringone (AS), whereafter the VirG protein triggers the transcriptional activation of the remaining *vir* loci (Winans, 1992). This in turn leads to production of the VirD2 endonuclease, which assisted by the VirD1 protein makes site-specific nicks within the 25-bp border repeats of the T-DNA (Scheiffele *et al.,* 1995, Pansegrau and Lanka, 1996). After border nicking VirD2 remains covalently linked to the 5'-end, of the T-DNA lower strand via a specific tyrosyl residue. Possibly by displacement synthesis starting from the free 3'OH end, the lower strand (T-strand) with the 5' attached VirD2 protein is released and transferred to the plant via the pilus/pore structure made up of VirB proteins. Efficient transport to the plant cell nucleus of the T-complex is mediatd by nuclear localization sequences (NLS) present in the C-terminal part of VirD2. The T-strand is believed to be co-operatively coated by VirE2, a single-stranded DNA binding protein that also possesses nuclear localization sequences (Zupan *et al.,* 1996). VirE2 has been shown necessary for preserving the 3'-end of the T-DNA (Rossi *et al.,* 1996), thus, the "packaging" function of VirE2 may provide protection against nuclease degradation in the plant cell. Otherwise VirE2 is also important for efficient nuclear delivery of the T-strand (Ziemienowicz et al, 1999).

Export of the T-complex from the *Agrobacterium* cell thus occurs via a mechanism that resembles bacterial conjugation. Conjugative plasmids encode sets of genes responsible for two distinct processes. Firstly, DNA processing by which the DNA is nicked at a specific site in the origin of transfer *(oriT)* sequence by a relaxase and auxiliary proteins, forming the so-called relaxosome. Secondly, transfer of a single-stranded DNA which is released by rolling circle replication, to the recipient via a multiprotein pilus/pore structure (Lanka and Wilkins 1995). Some plasmids carry the *(mob)* genes necessary for DNA processing at *oriT,* but lack the transfer *(tra)* genes for building the transport pilus/pore. Such plasmids can be mobilized by other, conjugative plasmids *i*.*e*. they can use the transport structure of such conjugative plasmids for their own transfer to the recipient. Whether such a mobilizable plasmid is transferred by a conjugative plasmid is determined to a large extent by the "coupling factor" encoded by the conjugative plasmid (Cabezon et al, 1997). It has been proposed that coupling proteins interact with the relaxosome and mediate the transfer of the single-stranded nucleoprotein complex to the mating machinery. They share homology around two putative nucleotide-binding motifs and therefore they may form the molecular motor allowing the nucleoprotein complex to be transported to the recipient cell. Besides the lack of information currently available on certain steps, the similarities between T-DNA transfer and bacterial conjugation have increased during the last few years. Specifically, the Ti plasmid virulence machinery mediates the transfer of the broad host range IncQ plasmid RSF1010 to plant cells (Buchanan-Wollaston et al., 1987) and between agrobacteria (Beijersbergen et al., 1992). This DNA transfer has been shown to depend particularly on a functional *virB* operon and *virD4.* The *virB* genes have been shown to be essential for tumorigenesis (Berger and Christie, 1994) and their products have been described to be associated with the bacterial envelope and to determine a pilus structure (Beijersbergen et al, 1994; Fullner et al, 1996). The VirD4 protein has all the characteristics of a coupling protein. These findings match perfectly with the genetic requirements for mobilization of small plasmids like RSF1010 among agrobacteria. However, pilus formation by conjugative plasmids is dependent on the VirB-related conjugative proteins, but not on the VirD4-like protein (Pansegrau et al., 1996) as was found for the Vir-pilus (Fullner et al, 1996).

Our studies were focussed on the limited host range bacteriocinogenic plasmid CloDF13, which originates from *Enterobacter cloacae* (Tieze *et al.,* 1969). It belongs to a group of mobilizable, but non-self-conjugative plasmids, with a small size that can be maintained at high copy number in enterobacteria (Nijkamp et al, 1986). We have cloned eukaryotic marker genes on CloDF13 and tested whether this plasmid could be transferred from *Agrobacterium* to yeast and to plant cells. Our results show that CloDF13 transfer is possible to eukaryotic cells and relies on a functional *virB* operon but is independent of the virD operon.

In summary, indications were obtained that the Agrobacterium virulence system mediates the transfer of genetic material to plant cells by a mechanism resembling conjugation. The transfer intermediate was found to be a ssDNA-protein complex, which was formed after the action of a Vir-encoded relaxase (VirD2) at a specific site, the border repeat (Lessl and Lanka, 1994). A transmembrane VirB pilus/pore protein complex turned out to be responsible for transport of the DNA across the bacterial membranes into the recipient. This VirB structure not only mediated transfer from Agrobacterium to plants, but also to fungi and other bacteria (Beijersbergen et al, 1992; Bundock et al, 1995; De Groot et al, 1998). Finally, the Vir-system mobilized the T-DNA of the Ti plasmid, but also the promiscuous *IncQ* plasmid to recipient cells, provided that the latter plasmid had intact mobilization functions and the *oriT* sequence. The frequency of IncQ plasmid mobilization, however, was 100-fold less than of the natural T-DNA (Bravo-Angel et al, 1999). We provide for the first time evidence for the unexpected finding that the Vir-system can mediate efficient transfer of the limited host range, enterobacterial plasmid CloDF13 as well. Mobilization relies on the presence of the CloDF13 *oriT* sequence as well as its mobilization genes. From the Vir-system of the donor the *virD* operon including the *VirD4* gene can be deleted without affecting CloDF-transfer to yeast indicating that they are functionally replaced by CloDF13 mobilization functions. This contrasts with the mobilization of the *IncQ* plasmid by the Vir-system, in which case presence of the *VirD4* gene remains essential. Advantages of the use of CloDF13 derivatives as novel plant vectors are clear as they offer novel traits, *i*.*e*.: a) they are small, high copy number plasmids in *E*.*coli* and can therefore be easily manipulated b) their transfer to yeast and plants is very efficient in contrast to the transfer of IncQ plasmids c) derivatives with two *oriT* sequences in direct repeat will lead to the formation of "T-DNAs" lacking vector parts as is the case in the Ti plasmid d) characteristics of the mobilization proteins may be exploited to direct their use not only for nuclear transformation, but also for organel transformation; e) similarly they offer advantages for the use of CloDF13 as a vector for gene targeting by homologous and site specific recombination.

### MATERIALS AND METHODS

### Recombinant DNA Techniques

Unless specified, standard protocols were followed for plasmid DNA isolation, cloning, restriction enzyme analysis, PCR amplifications, DNA gel electrophoresis and DNA hybridization (Sambrook et *al.,* 1989). Total DNA from yeast was isolated using the method described by Holm *et al.* (1986).

### Plasmid Constructions

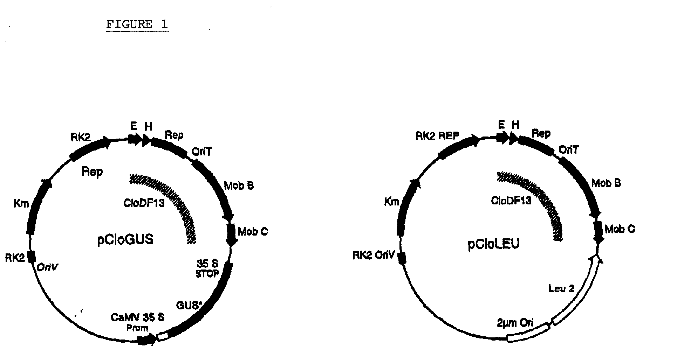

pCloLEU was constructed by insertion of: (i) a 4.6-kb *BamHI-SalI* region from plasmid CloDF13 (co-ordinates 1476-6624, anti-clock sense, Nijkamp *et al.*, 1986), containing the plasmid mobility region; (ii) a 3.4-kb *HindIII-SalI* fragment from plasmid pBEJ16 (Hadfield *et al.,* 1990) containing the 2ìm *ori*-STB region for replication and mitotic stability in *S*. *cerevisiae* plus *LEU2* as a yeast auxotrophic marker, into the IncP vector pRBJ (J. Escudero, unpublished) which is a pBin19 derivative from which one of the BglII fragments is deleted and replaced by the MCS of pUC19, containing a kanamycin resistance gene for selection in bacteria (see Figure 1). pCloGUS was constructed similarly as pCloLEU but in this case a 2.5-kb fragment containing the [CaMV 35S promoter-modified GUS-CaMV 35S terminator] gene cassette from plasmid pBG5 (Shen *et al.,* 1993) was used as marker gene for specific expression in plants (see Figure 1). *Agrobacterium* strains were electroporated with these plasmid constructs as described by Mozo & Hooykaas (1991).

### Bacterial and Yeast Strains

The *Agrobacterium tumefaciens* strains used in this work are listed in the Table 4. All bacterial strains contain the original C58 chromosomal background and either an octopine type pTiB6 plasmid with a wild-type vir-gene region or derivatives of it. The *Escherichia coli* strain used for cloning was DH5á (Sambrook *et al.,* 1989). *Saccharomyces cerevisiae* strain RSY12 (*MATa leu2-3,112 his3-11,15 ura3Δ ::HIS3)* was used as recipient cell in conjugation experiments with bacteria.

### Plasmid-DNA Transfer Assays

Conjugation assays between agrobacteria harboring pCloLeu and yeast were carried out as follows. The bacterial donor cells were grown for 2-3 days at 28°C on LC-agar (Hooykaas *et al.* 1979) medium plates in the presence of appropriate antibiotics (rifampicin, 10 mg/l; kanamycin, 100 mg/l; gentamycin, 80 mg/l; carbenicillin, 75 mg/l). From fresh cultures, a single colony was inoculated into 10 ml of LC liquid medium with the same antibiotic specification. Growth was allowed overnight at 28°C, shaking at 200 rpm to reach an OD₆₀₀ between1.0-1.5. Then bacteria were collected by centrifugation and washed with a 10 mM Mg SO₄ solution. Thereafter, bacteria were diluted to OD₆₀₀ ≈ 0.2 in two kind of minimal liquid media: (i) MM (Hooykaas *et al.,* 1979), which is regularly adjusted to pH7. (ii) IM [containing the same composition as the MM, plus 0.5 % (w/v) glycerol, 40 mM 2-(N-morpholino) ethanesulfonic acid (MES) and optional 0.2 mM AS], which is adjusted to pH 5.3. Bacteria in minimal liquid medium were further cultured for 8-10 hr at 28°C, shaking at 200 rpm, before being used for mating with the yeast cells. The yeast recipient cells were grown on YPD-agar (Sherman *et al.,* 1983) medium plates and a single colony was cultured overnight at 30°C in YPD-liquid medium. Yeast cells were then diluted 20 times in fresh YPD liquid medium and subsequently cultured for 8-10 hr at 30°C. Yeast cells were then collected by centrifugation and washed with either MM or with IM, then concentrated 10 times in the same medium before use. Subsequently, 50 µl of both the bacterial and yeast suspensions were gently mixed in an Eppendorf tube and finally placed on 0.45 ìm cellulose nitrate filters.

Bacteria-yeast conjugations were carried out either on MM-agar plates or on IM-agar plates, containing 5 mM glucose and the relevant aminoacids (leucine and uracil at 30 mg/l and histidine at 20 mg/l). After co-cultivation the filter with the cell mixture was immersed in 1 ml of PZ [physiological salt solution, 9 g/l (w/v) NaCl] and shaked vigorously for 10-15 min. Afterwards, 100 µl aliquots of this conjugation mixture were plated out on MY-agar medium (Zonneveld, 1986) plates containing 0.2 mM cefotaxim, to counterselect bacterial growth, and lacking leucine. The number of Leu⁺ transformed RSY12 colonies obtained in this way, after incubation for one week at 30°C, was taken as an estimate of the efficiency of successful plasmid transfer from agrobacteria to yeast. The output number of bacteria (donor cells) and yeast (recipient cells) was accurately determined by plating out dilutions of the conjugation mixture in the PZ solution: for bacteria on LC-agar medium containing the relevant antibiotics and for yeast on MY-agar medium containing the full set of required aminoacids. Plasmid DNA was isolated from the *Leu*^{*+*} transformed yeast colonies and used to transform *E*. *coli* cells for a proper characterisation by restriction analysis.

Plasmid pCloGUS transfer assays to plants were carried out as follows. Agrobacteria were grown and treated as specified above for the conjugation assays with yeast, except that after washing with the 10 mM Mg SO₄ solution the bacterial suspension was adjusted to an OD₆₀₀ ≈1.0 in MS-liquid medium (Murashige and Skoog, 1962) before use. Tobacco seedlings (Nicotiana tabacum, cv. Petit Havana line SR1) 7-to 10-days-old, from sterile *in vitro* germinated seeds in MS-agar medium, were used as plant-cell recipients. Routinely, twenty seedlings were immersed in 4 ml of bacterial suspension contained in plastic tubes and subjected to soft vacuum infiltration (- 0.5 atm with occasional gentle shaking) during fifteen min. Subsequently, the tobacco seedlings were quickly blotted on sterile paper and transferred to MS-agar medium plates containing 0.2mM AS. Bacteria and plant co-cultivation was then allowed for 3 days *in vitro* at 23°C in a growth chamber with a 16 hr light (2000 lux)/8 hr dark regime. The tobacco plantlets were then washed in sterile distilled water and subjected to a GUS histochemical assay as described (Escudero *et al*., 1995). The number of tobacco cells expressing GUS was then taken as an estimation of the efficiency of pCloGUS transfer from agrobacteria to the plant cell.

### Tumor Formation Assays

Agrobacteria were grown as described above on LC-agar medium with appropriate antibiotics. Bacterial cells were then resuspended in 10 mM MgSO₄ and adjusted to OD₆₀₀ ≈1 in Eppenforf tubes before use. Two-months-old *Nicotiana glauca* plants were infected by puncturing first in the stem with a sterile toothpick and subsequently applying 20 µl of the bacterial suspension to be tested into the wound. Routinely 3 infections were performed per plant and every test was repeated in at least two plants. Furthermore, independent infection experiments were carried out with different batches of plants. After infection of the plants with bacteria, plant cell proliferation (the so-called tumour formation) was due to the oncogenic nature of the native T-DNA. Plants were scored for tumour formation after 6 weeks post-infection.

### Results

### Trans-kingdom mobilization of Plasmid CloDF13 from Agrobacterium to yeast Requires vir-Gene Activation, Low Temperature and Long Mating Time

The CloDF13 plasmid is a small, non-conjugative plasmid, which can be mobilized among *E*. *coli* cells by the F plasmid and several other conjugative plasmids. The mobilization genes of CloDF13 are distinctly different from those of other mobilizable plasmids, such as the broad host range IncQ plasmid RSF1010. In addition, CloDF13 seems to encode a protein, MobB, related to the family of coupling proteins, such as pTi VirD4 and RP4 TraG. Hence, we were interested to find out whether CloDF13 could be mobilized by the pTi virulence system in interkingdom crosses and which Vir-proteins would be required for such a DNA transfer. Initially, we chose the yeast *Saccharomyces cerevisiae* as a recipient because of experimental convenience. Therefore, we constructed the plasmid pCloLEU (Figure 1), containing the mobilization region of CloDF13, the RK2 replicator for maintenance in agrobacteria, the yeast *LEU2* selection gene and the yeast 2µ replicator. We then did mating experiments between A. *tumefaciens* and *S. cerevisiae* RSY12, which is a haploid *Leu*⁻ strain, to test for plasmid transfer from bacteria to yeast. In this way indeed Leu⁺ yeast colonies were obtained indicative of pCloLEU transfer to yeast from Agrobacterium (LBA1100).

To investigate the transfer mechanism of the pCloLEU plasmid from agrobacteria to yeast, we assayed different values of four important parameters for Agrobacterium-mediated DNA transfer during our bacteria/yeast co-cultivation. Namely: (1) acidity of the medium (pH 5.3 versus pH 7); (2) temperature (23 °C versus 33 °C); (3) mating time (20 hr, 40 hr and 60 hr) and (4) presence or absence of the vir-gene inducer acetosyringone (AS). The results from Table 1 show that an acidic medium, low temperature and the inducer AS during the bacteria/yeast co-cultivation were essential for the recovery of Leu⁺ transformed yeast colonies. The length of mating time is also critical because high numbers of Leu⁺ yeast colonies were only observed after long co-cultivation. The estimated pCloLEU transfer frequency from the bacterial strain LBA1100 after 60 hr was 10⁻⁵ and this value decreased one order of magnitude per 20 hr shortening in co-cultivation time. A similar duration of co-cultivation was also necessary for T-DNA transfer from agrobacteria to yeast (Bundock et al, 1995). Hence, we concluded that pCloLEU transfer to yeast had all the characteristics of transport by the *Agrobacterium* Virulence system. For that, transcriptional activation of the *vir* regulon by the presence of AS is necessary and the particular mating complex in the agrobacterial donor, responsible for DNA/protein translocation, needs to be functionally established requiring the proper physical conditions.

### Role of Vir-proteins in the interkingdom CloDF13 mobilization

In order to establish which of the Vir-proteins are involved in the interkingdom transfer of CloDF13, we tested several vir-mutants for their ability to mobilize this plasmid to yeast. As it is shown in Table 2, transfer of pCloLEU did occur from agrobacteria with complete vir-systems (strains LBA1010, LBA1100 and GV3101 [pPM6000]). However, lack of *vir* genes (strain LBA288) resulted in no plasmid transfer. As a control, we created the plasmid pLEU, which is identical to pCloLEU but devoid of CloDF13 sequences. As expected, pLEU could not be transferred from agrobacteria to yeast cells (see below). The *virA* (LBA1142), *virB* (LBA1143) and *virG* (LBA1145) operons were essential for transfer to occur. This was expected since the VirA and VirG proteins are regulators of the expression of the vir-regulon, and it is believed that VirB proteins likely determine the mating structure. Mutants impaired in the gene for the single stranded DNA binding protein VirE2 (strains LBA1149 and ATΔvirE2) showed a tenfold lower frequency of transfer, as this was the case for T-DNA transfer to yeast (data not shown). The host-range gene *virF* (LBA1561) was not necessary for CloDF13 mobilization. As the CloDF13 mobilization region determines its own *oriT* sequence and the cognate relaxase protein, we expected that the pTi encoded border repeat specific relaxase VirD2 would not be necessary for CloDF13 transfer. This was indeed the case: strains with a non-polar insertion in *virD2* (LBA1147) or deletion of *virD2* (ATAvirD2) were equally mobilization-proficient as the wild-type strains. Similarly, CloDF13 transfer from *Agrobacterium* to yeast could be accomplished from bacteria with a mutation in the gene coding for the coupling factor VirD4 (strains LBA1148 and LBA1151). As VirD4 is essential for T-DNA transfer to yeast, it is probable that the protein encoded by the CloDF13 mobilization region, which resembles VirD4, can take over its function and interacts with the VirB complex. To confirm that such a characteristic is intrinsic for CloDF13-like plasmids, we also assayed a RSF1010 (IncQ) derivative plasmid. RSF1010 could not be transferred to yeast from the mutated *virD4* bacterial strain LBA1148 (data not shown).

### The CloDF13 mobB and mobC genes are Essential for Trans-kingdom Transfer

We constructed the plasmid pCloLEU by inserting the SalI-BamHI fragment (≈ 4.6 kb) from CloDF13 (Nijkamp et al., 1986), encompassing the mobilization region plus *oriT,* into a wide host range replicon (see Figure 1). In order to analyze the CloDF13 genetic elements that were required for the observed plasmid transfer from agrobacteria to yeast, a series of derivative plasmids was constructed by mutating stepwise the four CloDF13 genes present in pCloLEU: (i) pCloΔ ELEU, with a deletion of the gene *E* encoding the immunity protein; (ii) pCloΔEHLEU, with an additional deletion of the gene *H* encoding the cloacin excretion protein; (iii) pCloΔ EBLEU, with an additional deletion of the gene *mobB;* (iv) pCloΔECLEU, with an additional deletion of the gene *mobC*. The results, summarized in Table 3, indicated that neither gene E nor *H* is essential for Agrobacterium-mediated plasmid transfer to yeast. Plasmids pCloΔELEU and pCloΔEHLEU were transferred at high frequency, as their parental pCloLEU, from all transfer-proficient bacterial strains tested. Indeed the original plasmids pCloLEU, pCloΔELEU and pCloΔEHLEU, which were harboured by the bacterial donor, could be rescued from transformed *Leu*⁺ yeast cells after the mating experiments (data not shown). However, both CloDF13 *mobB* and *mobC* turned out to be essential for transfer, as in no case the plasmid pCloΔEBLEU or pCloΔECLEU was mobilized to yeast. Hence, genetic complementation of the CloDF13 *mob* genes did not occur by any agrobacterial *vir* counterpart, suggesting a strong specificity of the respective proteins for their cognate intermediate complex during conjugal transfer (see below).

### CloDF13 can also be Transferred to Plant Cells and does not Inhibit Agrobacterium Virulence

We were interested to find out whether CloDF13 transfer, as was described above from agrobacteria to yeast, would also occur to plant cells. Hence, the plasmid pCloGUS was constructed (Figure 1), which is similar to pCloLEU but carrying the gene for â-glucuronidase (gus) gene under plant expression signals. This *GUS* marker has been previously shown to be very sensitive in detecting T-DNA expressed in plants, both in tobacco as well as in maize cells (Rossi *et al.,* 1993; Shen *et al.,* 1993). After co-cultivation of agrobacteria with young tobacco *(Nicotiana tabacum*, line SR1) plants, the expression of the *GUS* gene encoded in the pCloGUS plasmid was assayed histochemically in the plant tissue. Blue staining, indicative of GUS activity, in the tobacco plantlets was clearly detected with the bacterial strains LBA1010 [pCloGUS, pTiB6] and LBA1100 [pCloGUS, pAL1100], both harbouring wild-type *vir* genes. This transient expression of the CloDF13-derivative plasmid was very abundant in the tobacco tissue. Therefore, we compared transfer of pCloGUS and transfer of T-DNA to tobacco cells. We assayed in parallel the bacterial strain LBA1100 [pCloGUS, pAL1100] with LBA1100 [pBG5, pAL1100], which carries the mentioned *GUS* gene as T-DNA marker in a RK2 replicon similar to the one used to construct pCloGUS. The efficiency in transfer of the pCloGUS plasmid was similar to that of T-DNA, judged from the number of plant cells showing GUS activity (data not shown). This result evidenced that transfer of pCloGUS and expression of the marker gene in the plant-cell nucleus took place at high efficiency.

As mentioned above and similarly to what was observed in the experiments with yeast as a recipient cell, the plasmid pCloGUS could be transferred to tobacco cells from strain LBA1010, which carries a wild-type T-DNA in its pTi plasmid. Importantly, the tumour formation in *Nicotiana glauca* plants infected with the strain LBA1010 was efficient and irrespective of the presence of plasmid pCloGUS (data not shown). Hence, contrary to what has been observed with agrobacteria containing RSF1010-derivative plasmids (Ward et al, 1991), there seems to be no interference between the transfer of the T-DNA and the CloDF13 complexes from agrobacteria to plant cells.

### Figure legend

Fig. 1 Plasmids pCloGUS and pCloLEU

### REFERENCES

**Berger, B.R. and P.J. Christie.** 1994. Genetic complementation analysis of the *Agrobacterium tumefaciens virB* operon: *virB2* through *virB11* are essential virulence genes. J.Bacteriol. **176**:3646-3660.
**Beijersbergen, A.,** A. Den Dulk-Ras, R.A. Schilperoort, and P.J.J. Hooykaas. 1992. Conjugative transfer by the virulence system of *Agrobacterium tumefaciens*. Science **256:** 1324-1327.
**Beijersbergen, A., S.J. Smith, and P.J.J. Hooykaas.** 1994. Localization and topology of VirB proteins of *Agrobacterium tumefaciens*. Plasmid **32**:212-218.
**Bonnard, G., B. Tinland, F. Paulus, E. Szegedi, and L. Otten.** 1989. Nucleotide sequence, evolutionary origin and biological role of a rearranged cytokinin gene isolated from a wide host range biotype III *Agrobacterium* strain. Mol.Gen.Genet. **216**:428-438.
**Bravo-Angel, A.M., B. Hohn, and B. Tinland.** 1998. The omega sequence of VirD2 is important but not essential for transfer of T-DNA by *Agrobacterium tumefaciens*. Mol.Plant Microbe Int. **11**:57-63.
**Bravo-Angel, A. M., V. Gloeckler, B. Hohn, and B. Tinland.** 1999. Bacterial conjugation proteinMobA mediates integration of complex DNA structures into plant cells. J. Bacteriol **181**:58-5765.
**Buchanan-Wollaston, V., J.E. Passiatore, and F. Cannon.** 1987. The *mob* and *oriT* mobilization functions of a bacterial plasmid promote its transfer to plants. Nature **328**:172-175.
**Bundock, P., A. Den Dulk-Ras, A. Beijersbergen, and P.J.J. Hooykaas.** 1995. Trans-kingdom T-DNA transfer from *Agrobacterium tumefaciens* to *Saccharomyces cerevisiae.* EMBO J. **14**:3206-3214.
**Cabezon, E., J.I. Sastre, and F. de la Cruz.** 1997. Genetic evidence of a coupling role for the TraG protein family in bacterial conjugation. Mol.Gen.Genet. **254**:400-406.
**Escudero, J., G. Neuhaus, and B. Hohn.** 1995. Intracellular *Agrobacterium* can transfer DNA to the cell nucleus of the host plant. Proc.Natl.Acad.Sci.USA **92**:230-234.
**Fullner, K.J., J.C. Lara, and E.W. Nester.** 1996. Pilus assembly by *Agrobacterium* T-DNA transfer genes. Science **273**:1107-1109.
**De Groot, M.J.A., P. Bundock, P.J.J. Hooykaas, and A.G.M. Beijersbergen.** 1998. *Agrobacterium tumefaciens*-mediated transformation of filamentous fungi. Nature Biotechnology **16**:839-842.
**Hadfield, C., Jordan, B.E., Mount, R.I., Pretorius, G.H.J. & Burak, E.** (1990) G418-resistance as a dominant marker and reporter for gene expression in *Saccharomyces cerevisiae*. *Curr*. *Genet.* **18**, 303-313.
**Holm, C., Meeks-Wagner, D.W., Fangman, W.L. & Botstain, P.** (1986) A rapid, efficient method for isolating DNA from yeast. *Gene* **42**,169-173.
**Hooykaas, P.J.J., Roobol, C. & Schilperoort, R.A.** (1979) Regulation of the transfer of Ti-plasmids of *Agrobacterium* tumefaciens. J. *Gen*. *Microbiol*. **110**, 99-109.
**Hooykaas, P.J.J. and R.A. Schilperoort.** 1992. Agrobacterium\ and plant genetic engineering. Plant Mol.Biol. **19**:15-38.
**Hooykaas, P.J.J. and A.G.M. Beijersbergen.** 1994. The virulence system of *Agrobacterium tumefaciens*. Ann.Rev.Phytopathol. **32**:157-179.
**Koekman, B.P., P.J.J. Hooykaas, and R.A. Schilperoort.** 1982. A functional map of the replicator region of the octopine Ti plasmid. Plasmid **7**: 119-132.
**Lanka, E. and B.M. Wilkins.** 1995. DNA processing reactions in bacterial conjugation. Ann.Rev.Biochem. **64**:141-169.
**Lessl, M. and E. Lanka.** 1994. Common mechanisms in bacterial conjugation and Ti-mediated T-DNA transfer to plant cells. Cell **77**:321-324.
**Mozo, T. and P.J.J. Hooykaas** (1991). Electroporation of megaplasmids into *Agrobacterium.* Plant Mol. Biol. 16, 917-918.
**Murashige, T. and F. Skoog.** 1962. A revised medium for rapid growth and bioassays with tobacco tissue cultures. Physiol. Plantarum **15**: 473-497.
**Nijkamp, H.J.J., De Lang, R., Stuitje, A.R., Van den Elzen, P.J.M., Veltkamp E. and Van Putten A.J.** (1986). The complete nucleotide sequence of the bacteriocinogenic plasmid CloDf13. Plasmid **16:** 135-160.
**Pansegrau, W. and E. Lanka.** 1996. Mechanisms of initiation and termination reactions in conjugative DNA processing. J. Biol.Chem. **271:** 13068-13076.
**Rossi, L., J. Escudero, B. Hohn, and B. Tinland.** (1993). Efficient and sensitive assay for T-DNA-dependent transient gene expression. Plant Mol. Biol. Reporter **11**: 220-229.
**Rossi, L., B. Hohn, and B. Tinland.** 1996. Integration of complete transferred DNA units is dependent on the activity of virulence E2 protein of *Agrobacterium tumefaciens*. Proc.Natl.Acad.Sci USA **93**:126-130.
**Sambrook, J., E.F. Fritsch, and T. Maniatis.** 1989. Anonymous molecular cloning. A laboratory manual. Second Edition. Cold Spring Harbor Laboratory, C.S.H. NY, USA.
**Scheiffele, P., W. Pansegrau, and E. Lanka.** 1995. Initiation of *Agrobacterium tumefaciens* T-DNA processing. J.Biol.Chem. **270**:1269-1276.
**Schrammeijer, B., J. Hemelaar, and P.J.J. Hooykaas.** 1998. The presence and characterization of a *virF* gene on *Agrobacterium vitis* Ti plasmids. Mol.Plant Microbe Int. **11**:429-433.
**Shen W.-H., Escudero J., Schläppi M., Ramos C., Hohn B. and Z. Koukolíková-Nicola** (1993). T-DNA transfer to maize cells: Histochemical investigation of β-glucuronidase activity in maize tissues. Proc. Natl. Acad. Sci. USA 90: 1488-1492.
**Sheng, J. and V. Citovsky.** 1996. *Agrobacterium*-plant cell DNA transport: have virulence proteins, will travel. Plant Cell **8**:1699-1710.
**Sherman F., Flink G.R. and Lawrence C.W.** (1983) Methods in yeast genetics, 2nd edn, p.61. Cold Spring Harbor Laboratory Press, Cold Spring Harbor, NY.
**Tieze G.A., Stouthamer A.H., Jansz H.S., Zandberg J. and van Bruggen E.F.J.** (1969) A bacteriocinogenic factor of Enterobacter cloacae. Molec. Gen. Genet. 106:48-65.
**Ward, J.E., E.M. Dale, and A.N. Binns.** 1991. Activity of the Agrobacterium\ T-DNA transfer machinery is affected by virB\ gene products. Proc.Natl.Acad.Sci.USA **88**:9350-9354.
**Winans, S.C.** 1992. Two-way chemical signaling in *Agrobacterium* -plant interactions. Microbiol.Rev. **56:**12-31.
**Ziemienowicz, A., D. Görlich, E. Lanka, B. Hohn, and L. Rossi.** 1999. Import of DNA into mammalian nuclei by proteins originating from a plant pathogenic bacterium. Proc.Natl.Acad.Sci.USA **96**:3729-3733.
**Zonneveld B.J.M.** (1986) Cheap and simple yeast media. J. Microbiol. Methods 4: 287-291.

**Table 1.**

| Transfer efficiencies of plasmid pCloLEU from wild-type agrobacterial strain LBA1100 to yeast depending on the *vir*-gene induction conditions, the temperature and the extent of time during mating | | | | | |
|---|---|---|---|---|---|
| Medium | Temperature (°C) | Time (hr) | *Titre Output* Donor | *(x10*^{*8*}*)*^{*a*} Recipient | Transfer Frequency^{b} |
| pH 5.3+AS | 23 | 20 | 0.2 | 0.8 | 2x10⁻⁸ |
| pH 5.3+AS | 23 | 40 | 0.9 | 1.0 | 4x10⁻⁷ |
| pH 5.3+AS | 23 | 60 | 1.0 | 0.9 | 3x10⁻⁶ |
| pH 5.3+AS | 33 | 60 | 0.2 | 1.0 | <10⁻⁸ |
| pH 5.3 | 23 | 60 | 0.9 | 1.0 | <10⁻⁸ |
| pH 7.0 | 23 | 60 | 3.0 | 1.1 | <10⁻⁸ |
| The strain LBA1100 [pCloLEU] was used as bacterial donor and the strain RSY12 was the yeast recipient. | | | | | |

| | | | | | |
|---|---|---|---|---|---|
| ^{a}Values represent number of bacterial donors and yeast recipient colonies per millilitre. | | | | | |
| ^{b} Estimated as the frequency of RSY12 Leu⁺ yeast colonies per output recipient. | | | | | |

**Table 2.**

| Transfer of the CloDF13-derivative plasmid pCloLEU from A. tumefaciens donor strains to the *S. cerivisiae* recipient strain RSY12 | | | |
|---|---|---|---|
| Bacterial Strain^{a} (*vir* mutation) | No. of RSY12 colonies (x10⁸)^{b} | No. Leu^{*} RSY12 colonies ^{b} | Frequency of Leu⁺ per recipient cell |
| LBA288 (No vir) | 2.2 | 0 | <2.2x10⁻⁸ |
| LBA1010 | 2.3 | 1175 | 0.5x10⁻⁵ |
| LBA1100 | 2.1 | 1200 | 0.5x10⁻⁵ |
| LBA2577 | 1.3 | 2700 | 2.0x10⁻⁵ |
| LBA1147 (3' *virD2*) | 1.5 | 2500 | 1.6x10⁻⁵ |
| LBA1148 (*virD4*) | 1.1 | 1400 | 1.4x10⁻⁵ |
| LBA1149 (*virE2*) | 2.0 | 154 | 0.7x10⁻⁶ |
| LBA2576(*virE2*) | 2.3 | 160 | 0.7x10⁻⁶ |
| LBA2561 (*virF*) | 1.5 | 1250 | 0.8x10⁻⁵ |

| | | | |
|---|---|---|---|
| ^{a}The bacterial strains LBA1010, LBA1100 and LBA2577 all contain a wild-type *vir* set of genes in their respective pTi plasmid. The bacterial strain LBA288 is a LBA1010 derivative in which the pTi plasmid was cured. All bacterial strains contained the plasmid pCloLEU and matings were performed at 23 °C, in pH 5.3 medium containing 0.2mM acetosyringone as described (see Materials and Methods). | | | |
| ^{b}Counting are values per millilitre of mating mixture. | | | |

**Table 3.**

| Effect of the different CloDF13 genetic components in the transfer efficiency of CloDF13-derivative plasmids from agrobacteria to yeast | | | |
|---|---|---|---|
| Bacterial Strain^{a} | No. of RSY12 colonies (x10⁸)^{b} | No. Leu⁺ RSY12 colonies^{b} | Frequency of Leu⁺ per recipient cell LBA1100 |
| [pJleu] | 1.8 | 0 | <1.8x10⁻⁸ |
| LBA1100 [pCloÄEleu] | 3.0 | 360 | 1.2x10⁻⁶ |
| LBA1100 [pCloÄEHleu] | 1.8 | 3200 | 1.7x10⁻⁵ |
| LBA1100 [pCloÄEBleu] | 3.0 | 0 | <3.0x10⁻⁸ |
| LBA1100 [pCloÄECleu] | 3.2 | 0 | <3.2x10⁻⁸ |
| LBA1148 [pCloÄEleu] | 3.0 | 400 | 1.3x10⁻⁶ |
| LBA1148 [pCloÄEHleu] | 1.7 | 2400 | 1.4x10⁻⁵ |
| LBA1148 [pCloÄEBleu] | 3.5 | 0 | <3.5x10⁻⁸ |
| LBA1148 [pCloÄECleu] | 2.5 | 0 | <2.5x10⁻⁸ |

**Table 4.**

| *Agrobacterium* strains used | | | |
|---|---|---|---|
| Strain | Chromosomal background | Ti Plasmid | Reference |
| LBA288 1979 | C58 | Cured of Ti, no *vir* | Hooykaas et al. |
| LBA1010 1982 | C58 | wild-type *vir* pTiB6 | Koekman et al. |
| LBA1100 | C58 | pAL1100, i.e. pTiB6 ΔTₗ, ΔTᵣ, Δ*tra*, *Δocc* | Beijersbergen et al. 1992 |
| LBA1142 | C58 | pAL1100 (*virA::Tn3Hoho1*) | idem |
| LBA1143 | C58 | pAL1100(*virB4::Tn3Hohol*) | idem |
| LBA1145 | C58 | pAL1100 (*virG::Tn3Hoho1*) | idem |
| LBA1147 | C58 | pAL1100 (*3'virD2::Tn3Hoho1*) | idem |
| LBA1148 | C58 | pAL1100 (*virD4::Tn3Hoho1*) | idem |
| LBA1149 | C58 | pAL1100 (*virE2::Tn3Hoho1*) | idem |
| LBA1151 | C58 | pAL1100 (*5'virD2::Tn3Hoho1*) | idem |
| LBA1561 | C58 | pAL1100 (ΔvirF) | Schrammeijer et al., 1998 |
| LBA2577 | C58 | pPM6000, i.e. pTiAch5 ΔTₗ, ΔTᵣ | Bonnard et al., 1989 |
| LBA2576 | C58 | pPM6000 (Δ*virE2*) | Rossi et al., 1996 |
| LBA2584 | C58 | pPM6000 (*ΔvirD2*) | Bravo-Angel et al., 1998 |

## Claims

1. A method for transferring genetic material which is not a typical T-DNA surrounded by Agrobacterium T-borders from an Agrobacterium virulence system to a eukaryotic host cell, comprising providing said genetic material on a mobilisable plasmid, capable of forming a relaxosome, bringing said mobilisable plasmid in an Agrobacterium having at least the activity of the transfer genes of Agrobacterium not present on said mobilisable plasmid, whereby the necessary gene products providing the same or similar activity as a functional VirB operon are also present and cocultivating said agrobacterium with said eukaryotic host cell.

2. A method according to claim 1, whereby said mobilisable plasmid comprises a functional *oriT* and a sequence encoding VirD-like mobilization activity as well as a sequence encoding a VirD4-like coupling factor.

3. A method according to claim 1 or 2, whereby said mobilisable plasmid comprises a functional oriT, but whereby VirD-like mobilization functions and the VirD4-like coupling factor are provided in trans.

4. A method according to any one of claims 1-3, whereby said mobilisable plasmid is derived from a group of mobilisable plasmids present in enterobacteria, which plasmids are non-conjugative.

5. A method according to claim 4, wherein said group of mobilisable plasmids comprises small plasmids which can be maintained in high copy number in enterobacteria.

6. A method according to claim 5, wherein said group of enterobacteria comprises E.coli.

7. A method according to any one of the aforegoing claims, wherein said mobilisable plasmid is derived from CloDF13.

8. A method according to any one of the afore going claims whereby said mobilisable plasmid is produced and or multiplied in an enterobacterium.

9. A method according to claim 8, wherein said enterobacterium is E.coli.

10. A mobilisable plasmid comprising genetic material to be transferred into a eukaryotic cell by Agrobacterium transfer, said mobilisable plasmid further comprising a functional *oriT,* sequences encoding functional virD-like mobilisation products, a VirD4-like coupling factor, and sequences encoding functional virB-like activity.

11. A mobilisable plasmid according to claim 10, which encodes the Mob functions of CloDF13.

12. A mobilisable plasmid according to claim 10 or 11, which is derived from a group of mobilisable plasmids present in enterobacteria, which plasmids are non-self-conjugative.

13. A mobilisable plasmid according to claim 12, which is derived from CloDF13.

14. Use of a plasmid according to any one of claims 10-13 for the transfer of genetic material to eukaryotic cells.

15. Use according to claim 14, wherein said transfer is to the nucleus or an organelle of said eukaryotic cell.

16. Use according to claim 14 or 15, wherein said eukaryotic cell is a plant cell.

17. Use according to claim 14 or 15, wherein said eukaryotic cell is a yeast cell and/or a fungal cell.
